# EUROPEAN PATENT SPECIFICATION

(11) **EP 3 141 906 B1**
(45) Date of publication and mention of the grant of the patent: **27.06.2018**
(21) Application number: 16185472.4
(22) Date of filing: 24.08.2016
(51) Int. Cl.: G01N 33/543, G01N 35/00

(54) **IMMUNE MEASURING APPARATUS AND IMMUNE MEASURING METHOD**
IMMUNMESSVORRICHTUNG UND IMMUNMESSVERFAHREN
APPAREIL DE MESURE IMMUNITAIRE ET PROCÉDÉ DE MESURE IMMUNOLOGIQUE

(30) Priority: 31.08.2015 JP 2015171112
(43) Date of publication of application: 15.03.2017
(73) Proprietor: SYSMEX CORPORATION, Kobe-shi Hyogo 651-0073 (JP)
(72) Inventor: Kawamoto, Yutaka, Hyogo, 651-0073 (JP); Fukuju, Toshikatsu, Hyogo, 651-0073 (JP)
(74) Representative: Hoffmann Eitle

(56) References cited:
- US-A1- 2005 287 614
- US-A1- 2010 297 778
- US-A1- 2012 149 127

## Description

### BACKGROUND

The invention relates to an immune measuring apparatus configured to measure a test substance in a sample.

There is an immune complex transfer method as a technique to enhance the sensitivity of measurement by an immune measuring apparatus (see, for example, Patent Literature 1).

In the technique disclosed in Patent Literature 1, an immune complex containing a test substance and a labeled antibody is generated on a carrier by using antigen-antibody reaction. After a liberating reagent is added into a measurement specimen containing a bound body of the immune complex and the carrier, the immune complex liberated from the carrier is extracted from the measurement specimen. Thus, the labeled antibody non-specifically adsorbed to the carrier is removed. Then, measurement is performed based on a label contained in the extracted immune complex.

Although not disclosed in Patent Literature 1, as a method of extracting the immune complex liberated from the carrier from the measurement specimen, there is a method of aspirating a liquid phase from a reaction chamber in a state where carriers are collected in the reaction chamber. When a magnetic particle is used as the carrier, for example, the magnetic particle can be magnetically collected by causing magnetic force to act on the magnetic particle in the reaction chamber disposed at a liquid phase aspiration position.

Patent Literature 2 describes an analyzer for analyzing a target substance (immune complex) contained in a sample using a reagent containing magnetic particles. The analyzer performs a transfer process of the cuvette from a pre-magnetism collecting part to a movable installing part.

### PRIOR ART CODUMENTS

### Patent document

[Patent Literature 1] Japanese Patent Application Publication No. Hei 1-254868
[Patent Literature 2] US 2017/147127 A1

### SUMMARY

When the liquid phase is aspirated by magnetically collecting the magnetic particle in the reaction chamber disposed at the aspiration position as described above, it takes time before the magnetic particle is sufficiently magnetically collected after the reaction chamber is disposed at the aspiration position. For this reason, a wait time for magnetic collection of the magnetic particle increases the time required for sample processing. Therefore, it is desired to shorten the time required for sample processing.

The invention aims at shortening the time required for sample processing.

This aim is accomplished by the subject matter of the independent claims.

The dependent claims concern particular embodiments.

### [Advantageous Effects of Invention]

The invention can shorten the time required for sample processing.

### BRIEF DESCRIPTION OF DRAWINGS

Fig. 1 is a schematic diagram illustrating an overview of an immune measuring apparatus according to an embodiment.
Fig. 2 is a schematic diagram illustrating a configuration example of a transfer unit in the immune measuring apparatus according to the embodiment.
Fig. 3 is a schematic diagram illustrating another configuration example of the transfer unit in the immune measuring apparatus according to the embodiment.
Fig. 4 is a schematic plan view illustrating a first level of the immune measuring apparatus according to the embodiment.
Fig. 5 is a schematic plan view illustrating a second level of the immune measuring apparatus according to the embodiment.
Fig. 6 is a diagram for explaining an overview of measurement by the immune measuring apparatus illustrated in Figs. 3 and 4.
Fig. 7 is a perspective view illustrating a configuration example of the transfer unit in the immune measuring apparatus illustrated in Figs. 3 and 4.
Fig. 8 is a perspective view illustrating a configuration example of a first holder and a second holder in the transfer unit.
Fig. 9 is a plan view of the transfer unit illustrated in Fig. 8.
Fig. 10 is a side view of the transfer unit illustrated in Fig. 8.
Fig. 11 is a schematic plan view for explaining a positional relationship between a permanent magnet as a magnetic source and a first reaction chamber.
Fig. 12 is a longitudinal sectional view of the first holder in a state where the first reaction chamber is placed in the first holder.
Fig. 13 is a schematic longitudinal sectional view illustrating another configuration example of the magnetic source.
Fig. 14 is a flowchart for explaining a measurement processing operation by the immune measuring apparatus according to the embodiment.
Fig. 15 is a flowchart for explaining liquid phase aspirating and dispensing operations illustrated in Fig. 14.

### EMBODIMENTS

With reference to the drawings, an embodiment is described below.

### [Overview of Immune Measuring Apparatus]

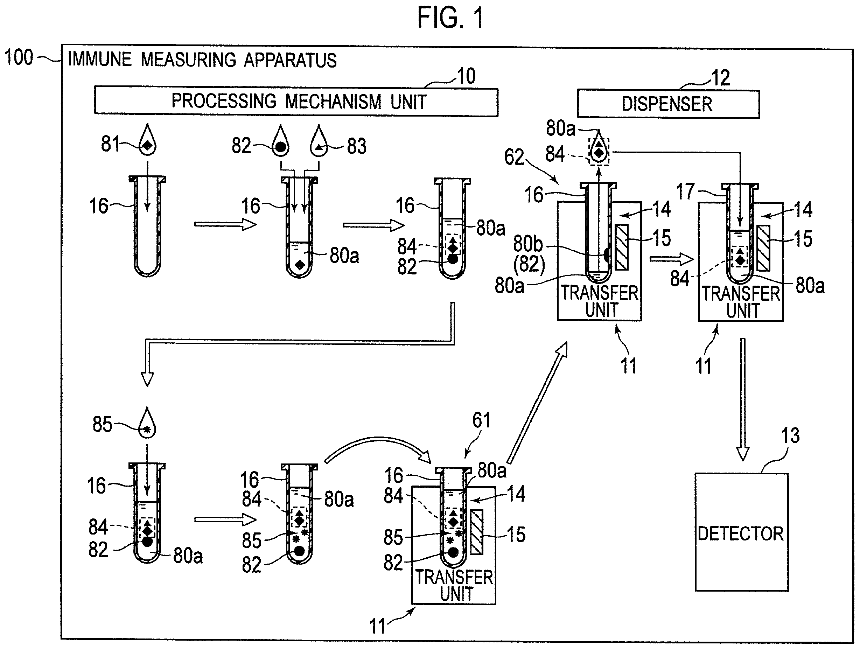

First, with reference to Fig. 1, an overview of immune measuring apparatus 100 according to an embodiment is described.

Immune measuring apparatus 100 is an apparatus configured to measure a test substance in a sample by using antigen-antibody reaction. Examples of the test substance include antigens or antibodies contained in blood, proteins, peptides, and the like. Immune measuring apparatus 100 acquires a serum as a sample for quantitative measurement or qualitative measurement of antigens or antibodies contained in the sample. The sample may be a plasma. Note that the antigen-antibody reaction includes not only reaction between antigens and antibodies but also reaction using a specific binding substance such as an aptamer. The aptamer is a nucleic acid molecule or peptide synthesized to specifically bind to a specific substance.

In this embodiment, immune measuring apparatus 100 performs separation processing of an immune complex by using an immune complex transfer method. In the immune complex transfer method, after an immune complex (bound body formed by antigen-antibody reaction) which contains a test substance and a labeled substance is formed on a solid-phase support, the immune complex and the solid-phase support are dissociated, and then the dissociated immune complex is separated from the solid-phase support. Thus, an unnecessary labeled substance non-specifically binding to the solid-phase support in the process of forming the immune complex on the solid-phase support is separated from the immune complex together with the solid-phase support. As a result, a noise level can be lowered compared with the case where measurement is performed without conducting the immune complex transfer method. Thus, a baseline of measured data can be lowered to enhance the sensitivity of immune measurement.

As illustrated in Fig. 1, immune measuring apparatus 100 includes processing mechanism unit 10, transfer unit 11, dispenser 12, and detector 13. In this embodiment, as described later, immune complex transfer is performed by dispensing liquid phase 80a containing immune complex 84 into second reaction chamber 17 from first reaction chamber 16. First and second reaction chambers 16 and 17 are each a cylindrical chamber having an opening on one end side and a closed bottom on the other end side, and can house a liquid such as a sample and a reagent. Such reaction chambers are disposable resin chambers, for example. In this case, a used reaction chamber can be discarded as it is.

Processing mechanism unit 10 has a function to execute processing required for immune measurement on the reaction chambers. In this embodiment, processing mechanism unit 10 performs processing of forming immune complex 84 containing test substance 81 in a sample and labeled substance 83 contained in a labeled reagent on magnetic particle 82 contained in a solid-phase reagent, in first reaction chamber 16. Also, processing mechanism unit 10 performs processing of liberating immune complex 84 from magnetic particle 82 with liberating reagent 85 after the formation of immune complex 84. Processing mechanism unit 10 may be configured to execute further processing other than the processing of forming immune complex 84 and of liberating immune complex 84.

Processing mechanism unit 10 may include one or more processing units according to the kinds and number of processing steps for the reaction chambers. One processing unit may carry out one kind of processing step or may carry out more kinds of processing steps.

For example, processing mechanism unit 10 may include a sample dispenser to dispense a sample into first reaction chamber 16. In this case, processing mechanism unit 10 can perform a step of dispensing the sample containing test substance 81. When processing mechanism unit 10 processes first reaction chamber 16 having in advance the sample dispensed therein, no sample dispenser needs to be provided in processing mechanism unit 10.

Moreover, processing mechanism unit 10 may include a reagent dispenser to dispense a reagent into first reaction chamber 16. In this case, processing mechanism unit 10 can perform a step of dispensing the solid-phase reagent containing magnetic particle 82, a step of dispensing the labeled reagent containing labeled substance 83, and a step of dispensing liberating reagent 85. When processing mechanism unit 10 processes first reaction chamber 16 having in advance such reagents dispensed therein, no reagent dispenser needs to be provided in processing mechanism unit 10.

Furthermore, processing mechanism unit 10 may include a reaction part to react a specimen in first reaction chamber 16 by heating the specimen. In this case, processing mechanism unit 10 can efficiently perform processing since the reaction of the specimen can be accelerated in a temperature environment suitable for the reaction during the processing of forming immune complex 84 or the processing of liberating immune complex 84. In such a case as where the reaction sufficiently proceeds without heating the specimen in first reaction chamber 16 or where processing mechanism unit 10 is entirely configured as a constant-temperature tank at a predetermined temperature, no reaction part needs to be provided in processing mechanism unit 10.

Various reagents to be dispensed into first reaction chamber 16 are liquid reagents, which are housed in different reagent containers according to type. The solid-phase reagent is a liquid reagent containing magnetic particle 82 in a liquid. The labeled reagent is a liquid reagent containing labeled substance 83 in a liquid. As described above, magnetic particle 82 serves as a carrier of immune complex 84.

The magnetic particle may be a particle containing a magnetic material as a base material for use in normal immune measurement. For example, a magnetic particle using Fe₂O₃ and/or Fe₃O₄, cobalt, nickel, phyllite, magnetite or the like as the base material can be used. Magnetic particle 82 may be coated with a binding substance for binding to test substance 81 or may bind to test substance 81 through a capture substance for binding magnetic particle 82 to test substance 81. The capture substance is an antigen, an antibody or the like, which binds to magnetic particle and test substance 81. In this case, a reagent containing the capture substance is dispensed into first reaction chamber 16.

Labeled substance 83 binds to test substance 81 by antigen-antibody reaction, and contains a label that can be measured by detector 13. Labeled substance 83 is not particularly limited as long as the labeled substance is an antibody containing a heretofore known label for use in immune measurement. In the case of using the capture substance, labeled substance 83 may bind to the capture substance. Examples of the label contained in the labeled substance include an enzyme, a fluorescent substance, a radioisotope, and the like. Examples of the enzyme include alkaline phosphatase (ALP), peroxidase, glucose oxidase, tyrosinase, acid phosphatase, and the like. As for the fluorescent substance, fluorescein isothiocyanate (FITC), green fluorescent protein (GFP), luciferin, and the like are available. As for the radioisotope, 125I, 14C, 32P, and the like are available. The enzyme is preferable as the label to be used for labeled substance 83 in this embodiment.

When the label is the enzyme, as for a substrate for the enzyme in labeled substance 83, a heretofore known substrate may be appropriately selected depending on the enzyme to be used. As for the substrate when alkaline phosphatase is used as the enzyme, for example, a chemiluminescent substrate such as CDP-Star (registered trademark), (4-chloro-3-(methoxyspiro {1,2-dioxetane-3,2'-(5'-chloro)tricyclo[3.3.1.13,7]decane}-4-yl) disodium phenyl phosphate) and CSPD (registered trademark) (3-(4-methoxyspiro{1,2-dioxetane-3,2-(5'-chloro)tricyclo[3.3. 1.13,7]decane}-4-yl) disodium phenyl phosphate; a luminescent substrate such as p-nitrophenyl phosphate, 5-bromo-4-chloro-3-indolyl phosphate (BCIP), 4-nitro blue tetrazolium chloride (NBT), and iodonitrotetrazolium (INT); a fluorescent substrate such as 4-methylumbelliferyl phosphate (4MUP); a chromogenic substrate such as 5-bromo-4-chloro-3-indolyl phosphate (BCIP), disodium 5-bromo-6-chloro-indolyl phosphate and p-nitrophenyl phosphate; and the like are available.

Liberating reagent 85 liberates immune complex 84 from magnetic particle 82 by releasing the binding between magnetic particle 82 and immune complex 84 containing test substance 81 and labeled substance 83. When magnetic particle 82 and test substance 81 bind to each other, liberating reagent 85 releases the binding between magnetic particle 82 and test substance 81. When magnetic particle 82 binds to test substance 81 through the capture substance, liberating reagent 85 may release the binding between magnetic particle 82 and the capture substance or the binding between test substance 81 and the capture substance. Liberating reagent 85 is selected according to the type of the binding between immune complex 84 and magnetic particle 82.

For example, when the binding between immune complex 84 and magnetic particle 82 is hapten-antihapten antibody binding, hapten or a hapten derivative can be used as the liberating reagent. When the binding between immune complex 84 and magnetic particle 82 is ion binding, a solution containing ions can be used as the liberating reagent. When the binding between immune complex 84 and magnetic particle 82 is ligand-receptor binding as separable binding, ligand or a ligand analog can be used as the liberating reagent. When the binding between immune complex 84 and magnetic particle 82 is lectin-carbohydrate chain binding as separable binding, carbohydrates can be used as the liberating reagent. When the binding between immune complex 84 and magnetic particle 82 is biotin-avidin binding, biotin can be used as the liberating reagent.

Transfer unit 11 can transfer the reaction chambers. Transfer unit 11 includes first holder 14 for holding first reaction chamber 16 and magnetic source 15. In this embodiment, transfer unit 11 can transfer first reaction chamber 16 to second position 62 while magnetically collecting magnetic particle 82, with magnetic source 15, in first reaction chamber 16 received in first holder 14 at first position 61. First position 61 is a position to start magnetic collection of magnetic particle 82 with magnetic source 15. Second position 62 is a position for dispenser 12 to aspirate liquid phase 80a containing immune complex 84 as described later. Transfer unit 11 may include one or more chamber transfer units, and the chamber transfer units may be configured to sequentially hand over the reaction chambers .

Transfer unit 11 may be capable of transferring first reaction chamber 16 or second reaction chamber 17 to the respective parts in processing mechanism unit 10, other than first position 61 and second position 62. When processing mechanism unit 10 includes processing units such as the sample dispenser, the reagent dispenser, and the reaction part, transfer unit 11 may be configured to be able to transfer the reaction chamber to a sample dispensing position of the sample dispenser, a reagent dispensing position of the reagent dispenser, and a hand-over position of the reaction chamber to the reaction part, for example. Moreover, transfer unit 11 may be configured to be able to transfer the reaction chamber to a hand-over position of second reaction chamber 17 to detector 13, other than first position 61 and second position 62.

Magnetic source 15 is disposed near first holder 14, and the magnetic force of magnetic source 15 can magnetically collect magnetic particle 82. The magnetic collection is collecting magnetic substances by the action of magnetic force. Magnetic source 15 allows the magnetic force to act on magnetic particle 82 in first reaction chamber 16 placed in first holder 14, thereby magnetically collecting magnetic particle 82 at a predetermined position such as the internal surface and bottom of first reaction chamber 16. As magnetic source 15, a permanent magnet or an electromagnet can be adopted, for example.

Magnetic source 15 may include magnetic sources arranged along a transfer path of first reaction chamber 16 from first position 61 to second position 62, or may include a magnetic source provided in first holder 14 and configured to move integrally with first holder 14. In the configuration where magnetic source 15 moves integrally with first holder 14, magnetic source 15 can be transferred together with first reaction chamber 16. Thus, the apparatus configuration can be simplified compared with the case where the magnetic source is placed along the transfer path of first reaction chamber 16.

Dispenser 12 has a function to aspirate liquid phase 80a containing liberated immune complex 84 in first reaction chamber 16 transferred to second position 62, and to dispense liquid phase 80a into second reaction chamber 17. Dispenser 12 aspirates a predetermined amount of liquid phase 80a from first reaction chamber 16 in a state where magnetic particle is magnetically collected at second position 62, and discharges aspirated liquid phase 80a into second reaction chamber 17 different from first reaction chamber 16. Thus, magnetic particle 82 magnetically collected as solid phase 80b in first reaction chamber 16 is separated from immune complex 84 contained in liquid phase 80a. When processing mechanism unit 10 includes the sample dispenser or reagent dispenser, the sample dispenser or reagent dispenser may function as dispenser 12 instead of providing dedicated dispenser 12. Therefore, dispenser 12 may be a part of processing mechanism unit 10.

Detector 13 has a function to detect a signal based on a label contained in immune complex 84 in liquid phase 80a dispensed into second reaction chamber 17. A detection method is not particularly limited as long as the detection is performed using an appropriate method according to the type of the label used in labeled substance 83. For example, when the label used in labeled substance 83 is an enzyme, measurement can be performed by measuring light, colors or the like generated by reaction between the enzyme and a substrate. In this case, a spectrophotometer, a luminometer or the like can be used as detector 13. Alternatively, when the labeled substance is a radioisotope, a scintillation counter or the like can be used as detector 13.

As described above, in this embodiment, magnetic source 15 is provided in transfer unit 11, and transfer unit 11 is configured to transfer first reaction chamber 16 to second position 62 while magnetically collecting magnetic particle 82, with magnetic source 15, in first reaction chamber 16 received in first holder 14 at first position 61. Thus, while transferring first reaction chamber 16 from first position 61 to second position 62, the transfer of first reaction chamber 16 does not have to be stopped to magnetically collect magnetic particle 82. Alternatively, stop time required for magnetic collection can be shortened even when the magnetic collection is performed while stopping first reaction chamber 16. As a result, since the stop time for the magnetic collection of magnetic particle 82 is eliminated or shortened, time required for sample processing can be shortened.

Note that Fig. 1 illustrates an example where the above configuration is applied to immune measuring apparatus 100 configured to perform separation processing of the immune complex using the immune complex transfer method. Alternatively, the above configuration may be applied to an immune measuring apparatus configured to perform immune measurement without conducting the immune complex transfer method.

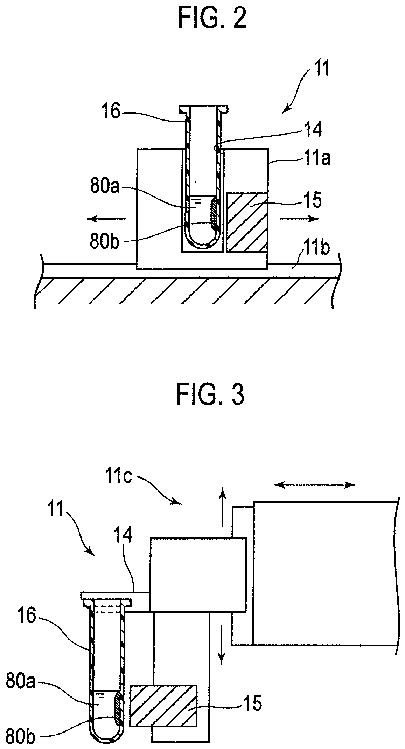

Figs. 2 and 3 illustrate configuration examples of transfer unit 11. In the configuration examples of Figs. 2 and 3, magnetic source 15 is provided in transfer unit 11 and transferred together with first reaction chamber 16

In the configuration example of Fig. 2, transfer unit 11 can move holding member 11a including first holder 14 along guide section 11b. In this configuration example, first holder 14 is an installation hole in which first reaction chamber 16 can be installed. Magnetic source 15 is provided in holding member 11a so as to be positioned near first holder 14, and is moved integrally with first holder 14.

In the configuration example of Fig. 3, transfer unit 11 can move first holder 14 by robot mechanism 11c that is movable in a desired direction such as a vertical direction and a horizontal direction. In this configuration example, first holder 14 is a catcher for holding the reaction chamber. Magnetic source 15 is provided at a position where the magnetic source can approach first reaction chamber 16 in a state where first reaction chamber 16 is held by first holder 14 in transfer unit 11, and is moved integrally with first holder 14.

### [Specific Configuration Example of Immune Measuring Apparatus]

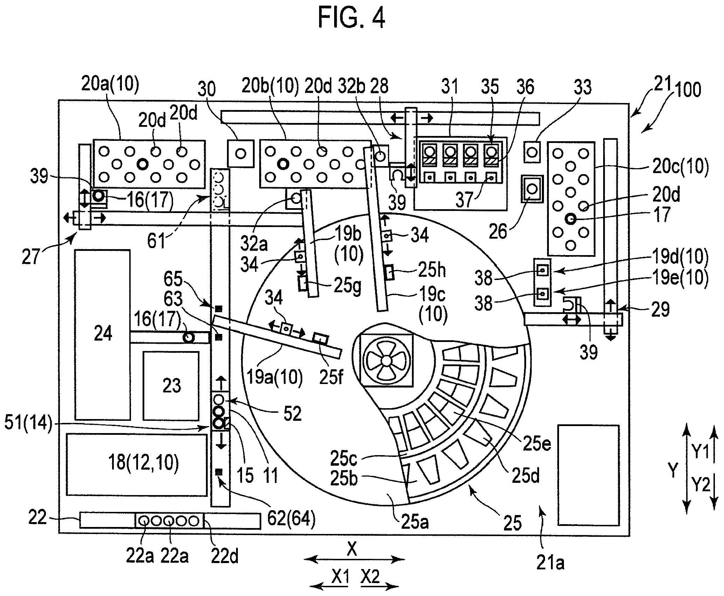

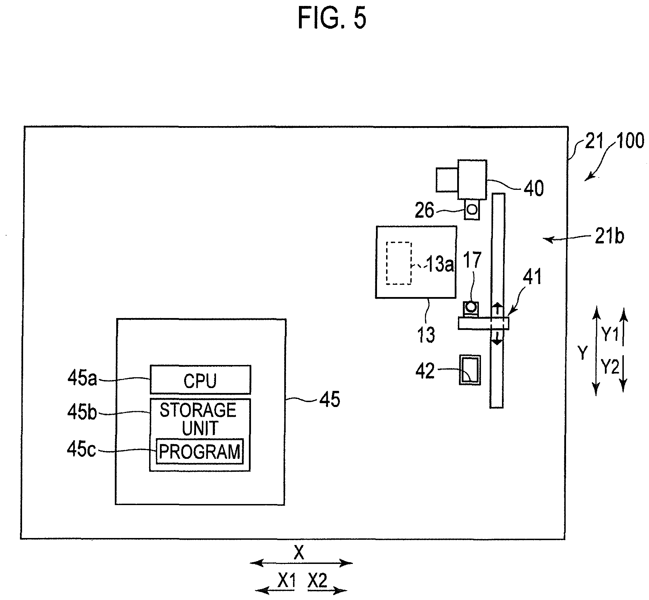

Next, a specific configuration example of immune measuring apparatus 100 is described in detail. The units in immune measuring apparatus 100 described above can be substantially realized with configurations as illustrated in Figs. 4 and 5, for example.

Immune measuring apparatus 100 includes processing mechanism unit 10, transfer unit 11, dispenser 12, detector 13, and controller 45 (see Fig. 5). In this configuration example, processing mechanism unit 10 includes sample dispenser 18, reagent dispensers 19a to 19e, and reaction parts 20a to 20c. Also, immune measuring apparatus 100 further includes housing 21, sample transport unit 22, chip supplier 23, chamber supplier 24, reagent holder 25, and inter-level transport unit 26. Furthermore, immune measuring apparatus 100 also includes transfer units 27, 28, and 29 configured to transport the reaction chamber to the units described above.

Housing 21 has a rectangular shape in a plan view. Housing 21 houses the units in immune measuring apparatus 100 therein. Housing 21 has a layered structure in which levels are provided in the vertical direction. Housing 21 includes first level 21a (see Fig. 4) and second level 21b (see Fig. 5) below first level 21a. The reaction chamber is transferred between first level 21a and second level 21b by inter-level transport unit 26. Note that housing 21 may include only one level. Hereinafter, for convenience, it is assumed that the horizontal direction along the long side of housing 21 is an X direction and the horizontal direction along the short side of housing 21 is a Y direction. Also, it is assumed that the vertical direction perpendicular to the X direction and the Y direction is a Z direction.

With reference to Fig. 4, description is given below of configurations of the units provided in first level 21a.

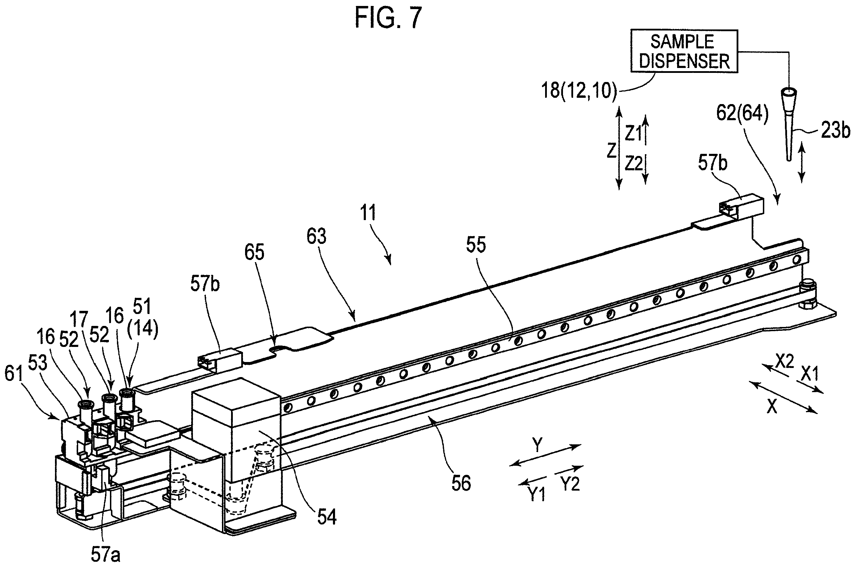

Sample transport unit 22 can transport rack 22b in which test tubes 22a, each housing a sample, are placed to a predetermined sample aspiration position. Chip supplier 23 can store a number of dispensing chips 23a (see Fig. 7) and supply dispensing chips 23a to sample dispenser 18. Dispensing chips 23a are each, for example, a hollow tip part capable of housing a predetermined amount of sample, and configured to be disposable. Dispensing can be performed while allowing only dispensing chip 23a to come into contact with the sample by aspirating the sample through dispensing chip 23a, housing the sample in dispensing chip 23a, and discharging the sample into the reaction chamber. The use of disposable dispensing chip 23a can prevent carry-over of the sample.

Chamber supplier 24 stores first reaction chamber 16 and second reaction chamber 17. Chamber supplier 24 can sequentially supply first reaction chamber 16 or second reaction chamber 17 one by one to transfer unit 11 at reaction chamber supply position 63. In this configuration example, reaction chambers of the same kind are used as first reaction chamber 16 and second reaction chamber 17. Note that first reaction chamber 16 and second reaction chamber 17 are simply referred to as the "reaction chamber" when differentiation therebetween is not required.

Sample dispenser 18 has a function to dispense the sample into first reaction chamber 16. Sample dispenser 18 can aspirate the sample in test tube 22a and dispense the aspirated sample into first reaction chamber 16 disposed at sample dispensing position 64.

In this embodiment, sample dispenser 18 may function as dispenser 12. In the configuration example of Fig. 4, sample dispenser 18 performs dispensing of the sample as well as aspiration and dispensing of liquid phase 80a.

Sample dispenser 18 dispenses the sample into first reaction chamber 16 transferred to sample dispensing position 64 by transfer unit 11, aspirates liquid phase 80a containing immune complex 84 from first reaction chamber 16 transferred to second position 62, and dispenses liquid phase 80a to second reaction chamber 17 transferred to sample dispensing position 64. Accordingly, sample dispenser 18 can function as dispenser 12. Therefore, immune measuring apparatus 100 can be reduced in size compared with the case where sample dispenser 18 and dispenser 12 are separately provided in immune measuring apparatus 100. Thus, time required to transfer first reaction chamber 16 can be shortened by reducing the travel distance of transfer unit 11.

Second position 62 may be the same as sample dispensing position 64. In this case, transfer unit 11 places first reaction chamber 16 at sample dispensing position 64 (= second position 62) for aspiration of liquid phase 80a containing immune complex 84, and places second reaction chamber 17 at sample dispensing position 64 for dispensing of liquid phase 80a containing immune complex 84. Thus, unlike the case where sample dispensing position 64 is different from second position 62, the sample dispenser 18 side does not have to be moved during the aspiration of liquid phase 80a from first reaction chamber 16 and the discharge of liquid phase 80a into second reaction chamber 17. As a result, the aspiration of liquid phase 80a and the discharge of liquid phase 80a can be quickly performed.

When sample dispenser 18 functions as dispenser 12, separate dispensing chips 23a is attached to sample dispenser 18 to perform the dispensing of the sample as well as the aspiration and dispensing of liquid phase 80a containing immune complex 84 liberated from magnetic particle 82. Thus, when sample dispenser 18 functions also as dispenser 12, too carry-over of the sample through dispensing chip 23a can be prevented.

In the configuration example of Fig. 4, transfer unit 11 includes first holder 14 and magnetic source 15, and can be moved linearly in the Y direction. Transfer unit 11 can be moved to first position 61, second position 62, reaction chamber supply position 63, sample dispensing position 64 (= second position 62), and reagent dispensing position 65. As described later, in the configuration example of Fig. 4, first position 61 is set as a hand-over position of the reaction chamber between reaction part 20a and transfer unit 11.

Transfer unit 11 is configured to be moved to second position 62, reaction chamber supply position 63, and sample dispensing position 64 (= second position 62). Thus, placement of empty second reaction chamber 17 at reaction chamber supply position 63 in transfer unit 11, aspiration of liquid phase 80a at second position 62 (sample dispensing position 64), and dispensing of liquid phase 80a and of the sample at sample dispensing position 64 can be performed only by moving transfer unit 11. Accordingly, various operations can be performed on the transfer path of transfer unit 11. As a result, the time required for the sample processing can be further shortened.

Reagent holder 25 includes cylindrical case 25a and annular reagent placement units 25b and 25c. Reagent holder 25 is a cold storage chamber configured to cool the reagent placed in insulating case 25a with a cooling mechanism.

Annular reagent placement units 25b and 25c are concentrically arranged and can be rotated independently of each other. Reagent placement unit 25b on the outer side can hold reagent containers 25d. Reagent placement unit 25c on the inner side can hold reagent containers 25e. Such reagent container 25d or 25e house R1 to R7 reagents to be described later. By the rotation of reagent placement units 25b and 25c, reagent containers 25d and 25e are positioned at respective predetermined reagent aspiration positions. In the upper surface of case 25a, three openable and closable aspiration holes 25f, 25g, and 25h are provided for aspiration by reagent dispensers 19a to 19c, respectively.

In the configuration example of Fig. 4, three reaction parts 20a to 20c are placed at fixed positions. Reaction parts 20a to 20c each include an unillustrated heater and a temperature sensor, and have a function to hold the reaction chamber and to react the specimen housed in the reaction chamber by heating the specimen. To be more specific, reaction parts 20a to 20c each include chamber holding holes 20d, and can heat the specimen housed in the reaction chamber placed in the chamber holding holes 20d to a predetermined temperature.

Reaction part 20a is provided near transfer unit 11. Reaction part 20a reacts the specimen in first reaction chamber 16 by heating the specimen. Transfer unit 11 receives first reaction chamber 16, in which immune complex 84 is liberated from magnetic particle 82 by liberating reagent 85, at first position 61 from reaction part 20a, and transfers received first reaction chamber 16 to second position 62. Thus, first reaction chamber 16 subjected to reaction processing by reaction part 20a can be quickly placed in transfer unit 11 and transferred to second position 62. As a result, the time required for the sample processing can be further shortened.

Between reaction parts 20a and 20b, magnetic collection port 30 is provided to hold the reaction chambers in the holding holes and to collect, with a magnet, magnetic particles in the specimen in the reaction chamber. In magnetic collection port 30, the reaction chamber can be handed over between transfer units 27 and 28.

Reaction part 20b is located between reaction part 20a and separator 31. Dispensing port 32a is provided between reaction part 20b and reagent holder 25. Dispensing port 32b is provided between reaction part 20b and separator 31.

Reaction part 20c is located on the X2 direction side with respect to separator 31. Between separator 31 and reaction part 20c, relay unit 33 and inter-level transport unit 26 are provided.

Reagent dispensers 19a to 19c perform processing of dispensing the reagent into first reaction chamber 16 or second reaction chamber 17. Reagent dispenser 19a can move pipette 34 for aspirating and discharging the reagent between aspiration hole 25f and reagent dispensing position 65. Pipette 34 aspirates the reagent from reagent container 25e in reagent holder 25, and dispenses the reagent into the reaction chamber transferred to reagent dispensing position 65.

Reagent dispenser 19b can move pipette 34 between aspiration hole 25g and dispensing port 32a. Pipette 34 aspirates the reagent from reagent container 25d in reagent holder 25, and dispenses the reagent into the reaction chamber transferred to dispensing port 32a.

Reagent dispenser 19c can move pipette 34 between aspiration hole 25h and dispensing port 32b. Pipette 34 aspirates the reagent from reagent container 25e in reagent holder 25, and dispenses the reagent into the reaction chamber transferred to dispensing port 32b.

Separator 31 has a function to execute BF separation processing to separate liquid phase 80a from solid phase 80b. Separator 31 includes processing ports 35 in each of which a reaction chamber can be placed. In each of processing ports 35, magnetic source 36 is provided for magnetic collection of magnetic particle 82 having immune complex 84 formed thereon. Magnetic source 36 is a permanent magnet, for example. Magnetic source 36 may be an electromagnet. In the configuration example of Fig. 4, four processing ports 35 are provided. Also, four cleaners 37 for aspirating liquid phase 80a and supplying a cleaning liquid are provided in total for four processing ports 35. Separator 31 uses cleaners 37 to aspirate the liquid phase in the reaction chambers and supply the cleaning liquid in a state where magnetic particle 82 having immune complex 84 formed thereon is magnetically collected. Thus, unnecessary components contained in the liquid phase can be separated and removed from the bound body of immune complex 84 and magnetic particle 82.

Relay unit 33 has a holding hole capable of holding the reaction chamber. In relay unit 33, the reaction chamber is handed over between transfer units 28 and 29.

Reagent dispensers 19d and 19e are arranged side by side, and each include reagent nozzle 38. Reagent dispensers 19d and 19e discharge R8 and R9 reagents into the reaction chambers from reagent nozzles 38, respectively.

Inter-level transport unit 26 has a holding hole capable of holding the reaction chamber. Inter-level transport unit 26 is moved up and down between first level 21a and second level 21b by elevator 40 to be described later.

Transfer units 27 to 29 each have a function to hold the reaction chamber and transport the reaction chamber to the units. Transfer units 27 to 29 are each an orthogonal robot mechanism capable of moving along three axes including two horizontal axes and one vertical axis, which are perpendicular to each other. Transfer units 27 to 29 have basically the same structure, and a heretofore known configuration can be adopted.

Transfer units 27 to 29 each include catcher 39 to hold the reaction chamber. Each of transfer units 27 to 29 can transport the reaction chamber to an arbitrary position within a range of movement by taking out the reaction chambers one by one with catcher 39.

Next, with reference to Fig. 5, description is given of configurations of the units provided in second level 21b.

Immune measuring apparatus 100 includes detector 13, elevator 40, chamber transport unit 41, chamber disposal opening 42, and controller 45 in second level 21b of housing 21.

Chamber transport unit 41 transports second reaction chamber 17 between inter-level transport unit 26 lowered to second level 21b, detector 13, and chamber disposal opening 42.

Detector 13 includes photodetector 13a such as a photomultiplier tube. Detector 13 uses photodetector 13a to acquire light generated in a reaction process between a labeled antibody and a luminescent substrate, which bind to the antigen of the sample subjected to various kinds of processing, thereby measuring the amount of antigens contained in the sample.

Controller 45 includes CPU 45a, storage unit 45b, and the like. CPU 45a functions as a controller of immune measuring apparatus 100 by executing control program 45c stored in storage unit 45b. Controller 45 controls operations of the units in immune measuring apparatus 100 described above.

### (Overview of Immune Measurement)

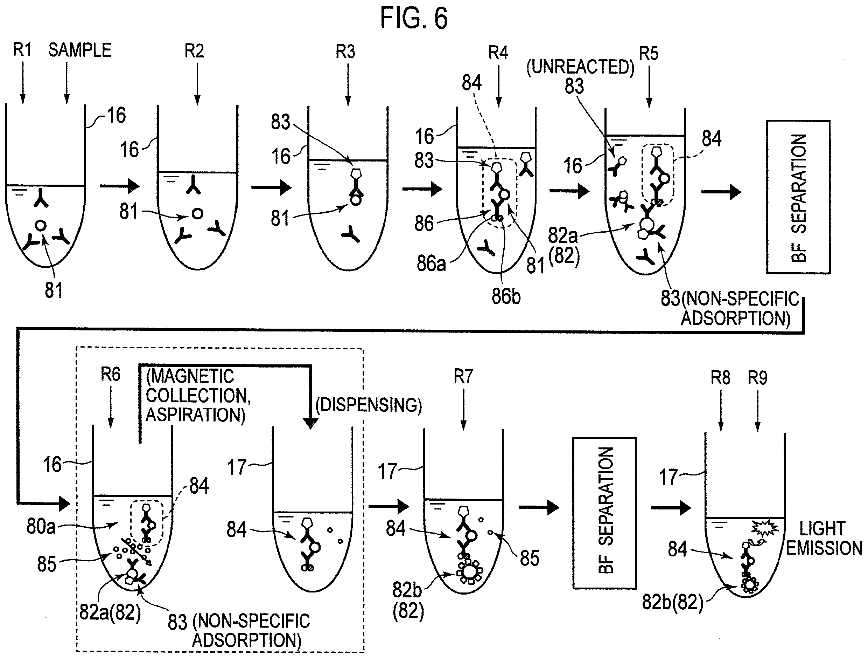

In the configuration examples illustrated in Figs. 4 and 5, immune measurement is performed using the R1 to R9 reagents, as illustrated in Fig. 6. Here, as an example of the immune measurement, description is given of an example where test substance 81 is a hepatitis B surface antigen (HBsAg).

First, a sample containing test substance 81 and the R1 reagent are dispensed into first reaction chamber 16. The R1 reagent is a reagent containing an alkaline substance for alkaline denaturation of the sample. The R1 reagent liberates an antigen present in the sample in a state of being already bound to an antibody from the antibody. Next, the R2 reagent is dispensed into first reaction chamber 16. The R2 reagent is a neutralization reagent containing an acidic substance to neutralize the alkaline substance in the sample after dispensing the R1 reagent. The R1 and R2 reagents are dispensed as preprocessing to liberate the antigen present in the sample in the state of being already bound to the antibody from the antibody. Depending on test substance 81, dispensing of the R1 and R2 reagents is not required.

Next, the R3 reagent is dispensed into first reaction chamber 16. The R3 reagent contains labeled substance 83, and reacts with and binds to test substance 81. In the example of Fig. 6, the labeled substance is an ALP (alkaline phosphatase) labeled antibody.

Next, the R4 reagent is dispensed into first reaction chamber 16. The R4 reagent contains capture substance 86, and reacts with and binds to test substance 81. Capture substance 86 contains first binding substance 86a for binding capture substance 86 to first magnetic particle 82a and second binding substance 86b for binding capture substance 86 to second magnetic particle 82b. First binding substance 86a and second binding substance 86b are substances that bind to the magnetic particle with different binding abilities.

For the binding between such binding substances and the magnetic particle, combinations of biotin and avidins, of hapten and an antihapten antibody, of nickel and a histidine tag, of glutathione and glutathione S-transferase, and the like can be used. Note that the "avidins" include avidin and streptavidin.

In this embodiment, capture substance 86 is an antibody (DNP/biotin antibody) modified with DNP (dinitrophenyl group) and biotin. More specifically, capture substance 86 modified with DNP (dinitrophenyl group) as first binding substance 86a and modified with biotin as second binding substance 86b.

Next, the R5 reagent is dispensed into first reaction chamber 16. The R5 reagent contains first magnetic particle 82a as magnetic particle 82. First magnetic particle 82a is a magnetic particle (anti-DNP antibody magnetic particle) having an anti-DNP antibody fixed. The anti-DNP antibody of the anti-DNP antibody magnetic particle that is antihapten reacts with and binds to DNP of capture substance 86 that is hapten. As a result, immune complex 84 containing test substance 81, labeled substance 83, and capture substance 86 is formed on first magnetic particle 82a.

Immune complex 84 formed on first magnetic particle 82a is separated from unreacted labeled substance 83 by primary BF separation processing. The primary BF separation processing removes unnecessary components such as unreacted labeled substance 83 from first reaction chamber 16. The primary BF separation processing is performed by separator 31 (see Fig. 4) .

After the primary BF separation processing, the R6 reagent is dispensed into first reaction chamber 16. The R6 reagent is liberating reagent 85. In the example of Fig. 6, DNP-Lys (DNP-Lysine) is used as liberating reagent 85. DNP-Lys reacts with and binds to the anti-DNP antibody magnetic particle that is first magnetic particle 82a. Therefore, when the R6 reagent is dispensed into first reaction chamber 16, the binding between DNP of capture substance 86 and first magnetic particle 82a and the binding between liberating reagent 85 (DNP-Lys) and first magnetic particle 82a compete against each other. As a result, immune complex 84 is dissociated from first magnetic particle 82a.

Liquid phase 80a containing immune complex 84 liberated by the R6 reagent is aspirated from first reaction chamber 16 by dispenser 12 (see Fig. 4) and dispensed into second reaction chamber 17. When sample dispenser 18 functions as dispenser 12, sample dispenser 18 aspirates and dispenses liquid phase 80a. Thus, liquid phase 80a containing immune complex 84 liberated from first magnetic particle 82a is moved from first reaction chamber 16 to second reaction chamber 17. First magnetic particle 82a remains in first reaction chamber 16 after the aspiration of liquid phase 80a containing immune complex 84. As a result, labeled substance 83 non-specifically binding to first magnetic particle 82a is separated from immune complex 84.

The R7 reagent is then dispensed into second reaction chamber 17 having immune complex 84 dispensed therein. The R7 reagent contains second magnetic particle 82b as magnetic particle 82. Second magnetic particle 82b binds to second binding substance 86b of capture substance 86. Second magnetic particle 82b is a magnetic particle (StAvi-bound magnetic particle) having fixed streptavidin that binds to biotin. Streptavidin in the StAvi-bound magnetic particle reacts with and binds to biotin that is second binding substance 86b. As a result, immune complex 84 containing test substance 81, labeled substance 83, and capture substance 86 binds to second magnetic particle 82b.

Immune complex 84 binding to second magnetic particle 82b and unnecessary components other than second magnetic particle 82b having immune complex 84 formed thereon are separated from each other by secondary BF separation processing. Thus, the unnecessary components are removed from second reaction chamber 17. The unnecessary components are, for example, liberating reagent 85 contained in liquid phase 80a, labeled substance 83 contained in liquid phase 80a together with immune complex 84 without binding to test substance 81, and the like. The secondary BF separation processing is performed by separator 31 (see Fig. 4) .

Thereafter, the R8 and R9 reagents are dispensed into second reaction chamber 17. The R8 reagent contains a buffer solution. Immune complex 84 bound to second magnetic particle 82b is dispersed into the buffer solution. The R9 reagent contains a chemiluminescent substrate. The buffer solution contained in the R8 reagent has a composition that accelerates reaction between the substrate and the label (enzyme) in labeled substance 83 contained in immune complex 84. Light is generated by the reaction between the label and the substrate, and detector 13 (see Fig. 5) measures the intensity of the generated light.

### [Structure of Transfer Unit]

Next, with reference to Figs. 7 to 12, a structural example of transfer unit 11 is described.

In the structural example of Fig. 7, transfer unit 11 includes holding member 53 for holding the reaction chambers, drive unit 54, and guide unit 55. Drive unit 54 includes a pulse motor, for example. Drive unit 54 may be a servomotor. An output shaft of drive unit 54 is connected to holding member 53 by transmission mechanism 56 such as a belt-pulley mechanism. Transmission mechanism 56 may be a rack and pinion mechanism, for example. Guide unit 55 includes a guide rail linearly extending along the Y direction in Fig. 4, for example. Guide unit 55 does not have to be linear, and may be formed to correspond to the transfer path of holding member 53. Holding member 53 is engaged with guide unit 55 in a movable state along guide unit 55. Holding member 53 is linearly moved in the Y direction along guide unit 55 by driving drive unit 54. First position 61, second position 62, reaction chamber supply position 63, sample dispensing position 64, and reagent dispensing position 65 are set on the linear path in the Y direction. Transfer unit 11 can dispose first and second reaction chambers 16 and 17 at first position 61, second position 62, reaction chamber supply position 63, sample dispensing position 64, and reagent dispensing position 65 by moving holding member 53.

Origin sensor 57a is provided at the end of transfer unit 11 in the Y direction. The position of holding member 53 is controlled based on the number of pulses from the origin position that is a detection position of origin sensor 57a. Also, chamber sensor 57b is provided at a predetermined position on transfer unit 11. Chamber sensor 57b is a transmissive optical sensor, for example. It is determined, based on a detection signal from chamber sensor 57b, whether or not the reaction chambers are set in holding member 53. Controller 45 performs operation control of transfer unit 11 and determination of whether or not the reaction chambers are set.

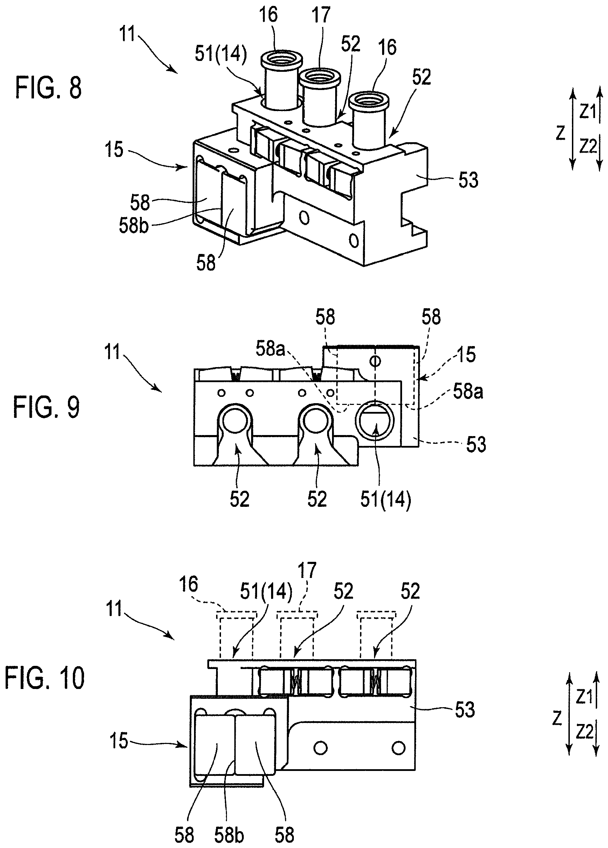

Figs. 8 to 12 illustrate a structural example of holding member 53.

As illustrated in Figs. 8 to 10, transfer unit 11 includes first holder 51 configured to hold first reaction chamber 16. Magnetic source 15 is installed on the side of or below first holder 51, for example. Thus, magnetic particle 82 can be magnetically collected along the inner surface or bottom of first reaction chamber 16. Therefore, during the aspiration of liquid phase 80a by dispenser 12, the aspiration tube or dispensing chip 23a in dispenser 12 is less likely to interfere with magnetically collected magnetic particle 82. Thus, some of magnetic particles 82 can be prevented from being aspirated together with liquid phase 80a. In the configuration example of Figs. 8 to 10, first holder 51 is an installation hole formed in holding member 53. Moreover, magnetic source 15 is disposed on the side of first holder 51. Magnetic source 15 may be installed below first holder 51.

In the configuration example of Figs. 8 to 10, magnetic source 15 includes permanent magnet 58 provided on the side of first holder 51. For efficient action of magnetic force on magnetic particle 82, it is preferable that permanent magnet 58 is provided at a position near first reaction chamber 16 set in first holder 51. In other words, permanent magnet 58 is disposed at a position slightly away from first reaction chamber 16 set in first holder 51 or at a position in contact therewith.

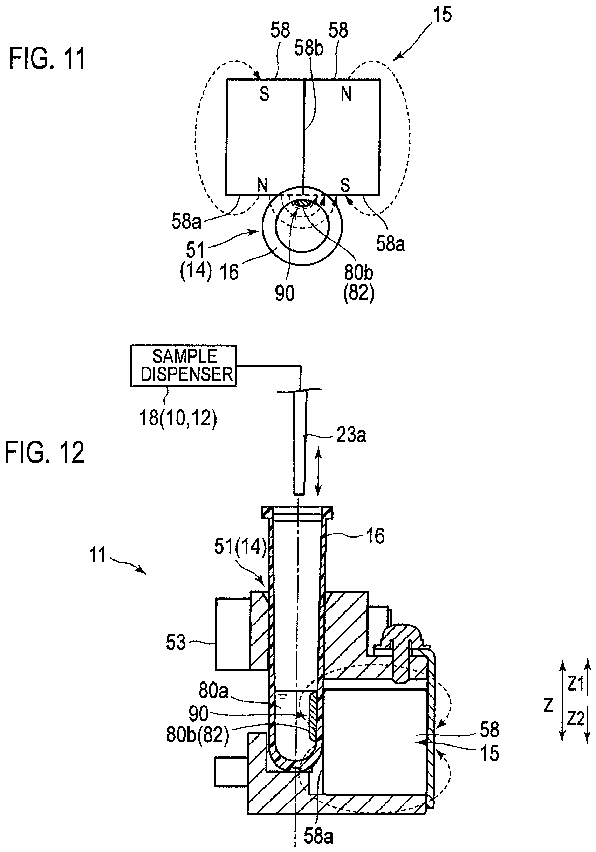

The shape and direction of permanent magnet 58 are not limited. For example, permanent magnet 58 can adopt a configuration in which magnetization surface 58a (see Fig. 11) is oriented toward the first holder 51 side and permanent magnet 58 extends along the longitudinal direction of first reaction chamber 16 set in first holder 51. In the configuration example of Figs. 8 to 10, the longitudinal direction of first reaction chamber 16 set in first holder 51 is the same as the vertical direction (Z direction). Permanent magnet 58 has a rectangular parallelepiped shape, and the longitudinal direction of permanent magnet 58 is the same as the longitudinal direction of first reaction chamber 16.

Thus, magnetization surface 58a can be set closer to first reaction chamber 16 set in first holder 51. As a result, stronger magnetic force can act on magnetic particle 82 in liquid phase 80a. To be more specific, as seen in the horizontal plane (see Fig. 11), the distance between magnetization surface 58a and the cylindrical inner surface of first reaction chamber 16 continuously changes. Thus, the strongest magnetic force acts at position 90 that minimizes the distance, and the more away from position 90, the weaker the magnetic force to act. On the other hand, as seen in the cross-section along the longitudinal direction of first reaction chamber 16 (see Fig. 12), position 90 that minimizes the distance extends along the inner surface of first reaction chamber 16. In other words, strong magnetic force can act in a wider range along the longitudinal direction of first reaction chamber 16. Therefore, permanent magnet 58 extending in the longitudinal direction (Z direction) of first reaction chamber 16 can allow stronger magnetic force to act on magnetic particle 82 in liquid phase 80a. As a result, more efficient magnetic collection can be achieved.

More than one permanent magnet 58 may be provided. In the configuration example of Fig. 11, a pair of permanent magnets 58 are provided. The pair of permanent magnets 58 include magnetization surfaces 58a with different polarities and facing toward first holder 51, and are arranged side by side in a direction perpendicular to the longitudinal direction of first reaction chamber 16. More specifically, magnetization surface 58a on first holder 51 side of one of permanent magnets 58 is the north pole, while magnetization surface 58a on first holder 51 side of the other permanent magnet 58 is the south pole. The pair of permanent magnets 58 are arranged in the horizontal direction perpendicular to the longitudinal direction of first reaction chamber 16. In other words, contact surface 58b between the pair of permanent magnets 58 extends along the longitudinal direction (see Fig. 10). Thus, the strongest magnetic force can be generated at a position between magnetization surfaces 58a of the pair of adjacent permanent magnets 58. Therefore, stronger magnetic force can be generated at position 90 that minimizes the distance between first reaction chamber 16 and magnetization surfaces 58a by arranging permanent magnets 58 such that the central axis of first reaction chamber 16 in the longitudinal direction is positioned in the same plane as that of contact surface 58b between the pair of permanent magnets 58. As a result, further efficient magnetic collection of magnetic particle 82 can be achieved.

Transfer unit 11 may be capable of holding more than one reaction chamber. In this case, transfer unit 11 can hold first and second reaction chambers 16 and 17 at the same time. In the configuration example of Figs. 8 to 10, transfer unit 11 further includes second holders 52 configured to hold the reaction chambers. Second holder 52 is formed in holding member 53 together with first holder 51. Second holder 52 is formed in a notch shape into which the reaction chamber can be inserted from above and side. Magnetic source 15 may be provided in first holder 14, and does not have to be provided in second holder 52.

When transfer unit 11 includes first and second holders 51 and 52, dispenser 12 (see Fig. 12) aspirates liquid phase 80a containing immune complex 84, which is liberated from magnetic particle 82, from first reaction chamber 16 set in first holder 51, and dispenses aspirated liquid phase 80a into second reaction chamber 17 set in second holder 52. Thus, during the aspiration of liquid phase 80a from first reaction chamber 16 and the dispensing of aspirated liquid phase 80a into second reaction chamber 17, first and second reaction chambers 16 and 17 do not need to be switched on transfer unit 11 side. Thus, the aspiration and dispensing of liquid phase 80a from and into the reaction chambers can be collectively performed. Therefore, the time required for sample processing can be further shortened.

Transfer unit 11 may include more than one second holder 52. In the configuration example of Figs. 8 to 10, transfer unit 11 includes two second holders 52. In this case, reagent dispenser 19a (see Fig. 4) dispenses a reagent into first reaction chamber 16 set in one of second holders 62, and dispenser 12 dispenses liquid phase 80a containing immune complex 84 liberated from magnetic particle 82 into second reaction chamber 17 set in the other second holder 52.

With such a configuration, even while using first holder 51 and one of second holders 52 for aspiration and dispensing of liquid phase 80a by dispenser 12, reagent dispensing can be concurrently performed by transferring another first reaction chamber 16 to reagent dispensing position 65 while holding first reaction chamber 16 by the other second holder 52. Therefore, when samples are continuously processed, another first reaction chamber 16 can be transferred during aspiration and dispensing of liquid phase 80a by dispenser 12. As a result, it is no longer required to interrupt transfer of another first reaction chamber 16 for aspiration and dispensing of liquid phase 80a by dispenser 12. Thus, the time required for sample processing can be further shortened.

### (Another Configuration Example of Magnetic Source)

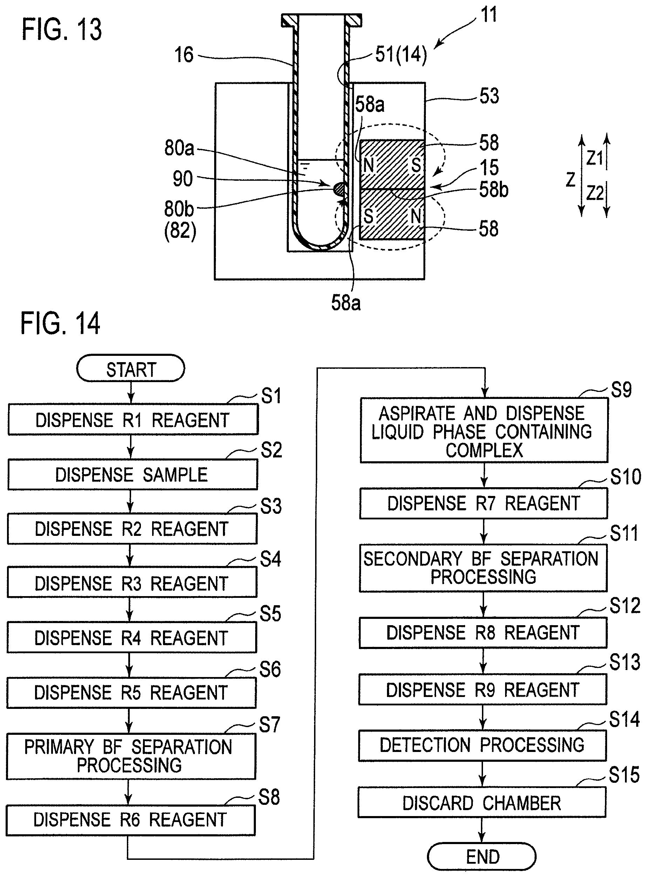

As illustrated in Fig. 13, a pair of permanent magnets 58 in magnetic source 15 may have magnetization surfaces 58a with different polarities and facing toward first holder 51, and may be arranged side by side along the longitudinal direction of first reaction chamber 16. More specifically, permanent magnets 58 are vertically stacked along the longitudinal direction of first reaction chamber 16. In this case, a region where the strongest magnetic force acts is an approximately point local region of position 90 when seen in the horizontal plane (see Fig. 11) or in the cross-section along the longitudinal direction of first reaction chamber 16 (see Fig. 13) . Therefore, in terms of magnetic collection efficiency, the configuration illustrated in Figs. 8 to 12 is preferable.

Besides the above, although not illustrated, magnetic source 15 may be disposed below first holder 51. In this case, magnetic particle 82 collected at the bottom of first reaction chamber 16 makes it difficult to aspirate liquid phase 80a to the bottom of first reaction chamber 16. Therefore, in order to reduce dilution of test substance 81 by minimizing the amount of liquid phase 80a, it is preferable to dispose magnetic source 15 on the side of first holder 51 as in the configuration example illustrated in Figs. 8 to 12, thereby collecting magnetic particle 82 on the inner surface of first reaction chamber 16. This is because liquid phase 80a can be aspirated to the bottom of first reaction chamber 16 even with a small amount of liquid phase 80a.

### (Explanation of Immune Measurement Processing Operation)

Next, with reference to Fig. 14, description is given of a measurement processing operation by immune measuring apparatus 100 illustrated in Figs. 4 and 5. In the following description, Fig. 14 is referred to for steps of the measurement processing operation, while Figs. 4 and 5 are referred to for the units in immune measuring apparatus 100. Controller 45 performs operation control of the measurement processing by immune measuring apparatus 100.

In Step S1, the R1 reagent is dispensed into first reaction chamber 16. To be more specific, first reaction chamber 16 is supplied to second holder 52 in transfer unit 11 from chamber supplier 24. Transfer unit 11 transfers first reaction chamber 16 to reagent dispensing position 65, and reagent dispenser 19a dispenses the R1 reagent into first reaction chamber 16 held by transfer unit 11.

In Step S2, the sample is dispensed into first reaction chamber 16. To be more specific, dispensing chip 23a is attached to sample dispenser 18, dispensing chip 23a being supplied by chip supplier 23 to aspirate the sample from test tube 22a. Sample dispenser 18 dispenses the aspirated sample into first reaction chamber 16 held by transfer unit 11 at sample dispensing position 64. After the dispensing, dispensing chip 23a is discarded into an unillustrated disposal opening. Sample dispenser 18 repeats a sample dispensing operation twice, including attaching dispensing chip 23a, aspirating the sample, dispensing the sample, and discarding dispensing chip 23a as a unit sequence. Thus, the sample is dispensed into first reaction chamber 16 in an amount twice as large as a unit amount that can be dispensed by dispensing chip 23a.

After the dispensing of the sample, transfer unit 11 transfers first reaction chamber 16 to first position 61. Transfer unit 27 takes first reaction chamber 16 out of second holder 52, and places first reaction chamber 16 in reaction part 20a. First reaction chamber 16 is maintained at a predetermined temperature for a predetermined period of time in reaction part 20a.

In Step S3, the R2 reagent is dispensed into first reaction chamber 16. To be more specific, transfer unit 27 takes first reaction chamber 16 out of reaction part 20a, and places first reaction chamber 16 in transfer unit 11. Transfer unit 11 transfers first reaction chamber 16 to reagent dispensing position 65, and reagent dispenser 19a dispenses the R2 reagent into first reaction chamber 16 held by transfer unit 11. After the dispensing of the R2 reagent, transfer unit 27 takes first reaction chamber 16 out of second holder 52 at first position 61, and places first reaction chamber 16 in reaction part 20a. First reaction chamber 16 is maintained at a predetermined temperature for a predetermined period of time in reaction part 20a.

In Step S4, the R3 reagent is dispensed into first reaction chamber 16. To be more specific, transfer unit 27 takes first reaction chamber 16 out of reaction part 20a, and places first reaction chamber 16 in dispensing port 32a. Transfer unit 28 transfers first reaction chamber 16 from dispensing port 32a to dispensing port 32b. Then, reagent dispenser 19c dispenses the R3 reagent into first reaction chamber 16 placed in dispensing port 32b. After the dispensing of the R3 reagent, transfer unit 28 transfers first reaction chamber 16 from dispensing port 32b to reaction part 20b. First reaction chamber 16 is maintained at a predetermined temperature for a predetermined period of time in reaction part 20b.

In Step S5, the R4 reagent is dispensed into first reaction chamber 16. To be more specific, transfer unit 28 transfers first reaction chamber 16 from reaction part 20b to dispensing port 32b. Then, reagent dispenser 19c dispenses the R4 reagent into first reaction chamber 16 placed in dispensing port 32b. After the dispensing of the R4 reagent, transfer unit 28 transfers first reaction chamber 16 from dispensing port 32b to reaction part 20b. First reaction chamber 16 is maintained at a predetermined temperature for a predetermined period of time in reaction part 20b.

In Step S6, the R5 reagent is dispensed into first reaction chamber 16. To be more specific, transfer unit 28 transfers first reaction chamber 16 from reaction part 20b to dispensing port 32a. Then, reagent dispenser 19b dispenses the R5 reagent into first reaction chamber 16 placed in dispensing port 32a. After the dispensing of the R5 reagent, transfer unit 28 transfers first reaction chamber 16 from dispensing port 32a to reaction part 20b. First reaction chamber 16 is maintained at a predetermined temperature for a predetermined period of time in reaction part 20b.

In Step S7, separator 31 performs primary BF separation processing. To be more specific, transfer unit 28 takes first reaction chamber 16 out of reaction part 20b, and places first reaction chamber 16 in processing port 35 in separator 31. Separator 31 performs the primary BF separation processing on the specimen in first reaction chamber 16, thereby removing the liquid phase and cleaning the solid phase with a cleaning liquid.

In Step S8, the R6 reagent is dispensed into first reaction chamber 16. To be more specific, transfer unit 28 transfers first reaction chamber 16 from separator 31 to dispensing port 32a. Then, reagent dispenser 19b dispenses the R6 reagent into first reaction chamber 16 placed in dispensing port 32a. After the dispensing of the R6 reagent, transfer unit 27 transfers first reaction chamber 16 from dispensing port 32a to reaction part 20a. First reaction chamber 16 is maintained at a predetermined temperature for a predetermined period of time in reaction part 20a.

In Step S9, liquid phase 80a containing immune complex 84 is aspirated from first reaction chamber 16. To be more specific, empty second reaction chamber 17 is supplied to second holder 52 in transfer unit 11, and transfer unit 27 sets first reaction chamber 16 in first holder 51 in transfer unit 11. In this event, magnetic source 15 magnetically collects magnetic particles in first reaction chamber 16. Then, sample dispenser 18 aspirates liquid phase 80a from first reaction chamber 16 held by first holder 51 in transfer unit 11. After the aspiration, sample dispenser 18 dispenses liquid phase 80a into second reaction chamber 17 held by transfer unit 11. Thereafter, first reaction chamber 16 is discarded. Second reaction chamber 17 is set in reaction part 20a by transfer unit 27. Second reaction chamber 17 is maintained at a predetermined temperature for a predetermined period of time in reaction part 20a. The processing of aspirating and dispensing liquid phase 80a in Step S9 is described in detail later.

In Step S10, the R7 reagent is dispensed into second reaction chamber 17. To be more specific, transfer unit 27 transfers second reaction chamber 17 from reaction part 20a to dispensing port 32a. Then, reagent dispenser 19b dispenses the R7 reagent into second reaction chamber 17 placed in dispensing port 32a. After the dispensing of the R7 reagent, transfer unit 28 transfers second reaction chamber 17 from dispensing port 32a to relay unit 33. Thereafter, transfer unit 29 transfers second reaction chamber 17 from relay unit 33 to reaction part 20c. Second reaction chamber 17 is maintained at a predetermined temperature for a predetermined period of time in reaction part 20c.

In Step S11, separator 31 performs secondary BF separation processing. To be more specific, transfer unit 29 transfers second reaction chamber 17 from reaction part 20c to relay unit 33, and transfer unit 28 transfers second reaction chamber 17 from relay unit 33 to separator 31. Contents of the secondary BF separation processing are the same as those of the primary BF separation processing in Step S7.

In Step S12, the R8 reagent is dispensed into second reaction chamber 17. To be more specific, transfer unit 28 transfers second reaction chamber 17 from dispensing port 32a to relay unit 33. Then, transfer unit 29 transfers second reaction chamber 17 from relay unit 33 to reagent dispenser 19d. Thereafter, reagent dispenser 19d dispenses the R8 reagent into second reaction chamber 17.

In Step S13, the R9 reagent is dispensed into second reaction chamber 17. To be more specific, transfer unit 29 transfers second reaction chamber 17 from reagent dispenser 19d to reagent dispenser 19e. Then, reagent dispenser 19e dispenses the R9 reagent into second reaction chamber 17. After the dispensing of the R9 reagent, transfer unit 29 transfers second reaction chamber 17 to reaction part 20c. Second reaction chamber 17 is maintained at a predetermined temperature for a predetermined period of time in reaction part 20c.

In Step S14, measurement processing of immune complex 84 is performed. To be more specific, transfer unit 29 transfers second reaction chamber 17 from reaction part 20c to inter-level transport unit 26. Inter-level transport unit 26 transfers second reaction chamber 17 from first level 21a to second level 21b. Chamber transport unit 41 transfers second reaction chamber 17 from inter-level transport unit 26 to detector 13. Detector 13 measures the intensity of light generated by reaction between the label and the substrate. The measurement result obtained by detector 13 is outputted to an unillustrated controller.

After the completion of the detection, chamber transport unit 41 transports second reaction chamber 17 subjected to the measurement from detector 13 to chamber disposal opening 42, and discards second reaction chamber 17 subjected to the measurement.

Thus, immune measuring apparatus 100 performs the measurement processing operation.

### (Processing of Aspirating and Dispensing Liquid Phase Containing Complex)

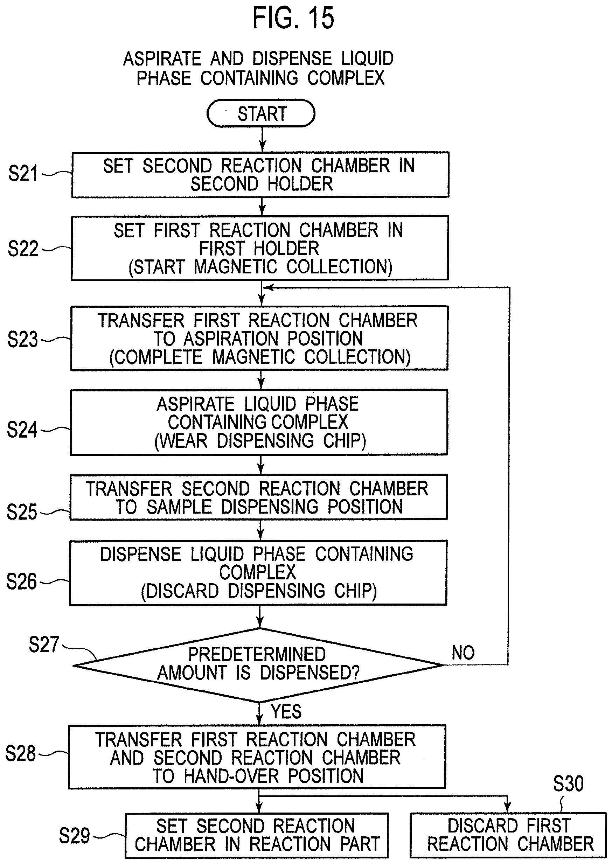

Next, with reference to Fig. 15, description is given of details about the processing of aspirating and dispensing liquid phase 80a containing immune complex 84 illustrated in Step S9 of Fig. 14. In the following description, Fig. 15 is referred to for steps of the processing of aspirating and dispensing liquid phase 80a, while Fig. 4 is referred to for the units in immune measuring apparatus 100. Controller 45 performs operation control of the processing of aspirating and dispensing liquid phase 80a.

In Step S21 of Fig. 15, transfer unit 11 places second holder 52 at reaction chamber supply position 63, and receives empty second reaction chamber 17 in second holder 52 from chamber supplier 24.

In Step S22, transfer unit 11 moves to first position 61, and transfer unit 27 takes first reaction chamber 16 out of reaction part 20a, and sets first reaction chamber 16 in first holder 51 in transfer unit 11. When first reaction chamber 16 is set in first holder 51, magnetic source 15 starts magnetic collection of magnetic particle in first reaction chamber 16. Note that Steps S21 and S22 may be executed in reverse order.

In Step S23, transfer unit 11 places first holder 51 at sample dispensing position 64 (= second position 62). The magnetic collection by magnetic source 15 proceeds while moving first holder 51 from first position 61 to sample dispensing position 64, and the magnetic collection of magnetic particle 82 is completed when first holder 51 reaches sample dispensing position 64.

In Step S24, new dispensing chip 23a from chip supplier 23 is attached to sample dispenser 18, and sample dispenser 18 aspirates a predetermined amount of liquid phase 80a from first reaction chamber 16 held by transfer unit 11 at sample dispensing position 64 (= second position 62).

In Step S25, transfer unit 11 places second holder 52 configured to hold empty second reaction chamber 17 acquired in Step S21 at sample dispensing position 64.

In Step S26, sample dispenser 18 dispenses the aspirated predetermined amount of liquid phase 80a into second reaction chamber 17 held by transfer unit 11 at sample dispensing position 64. After the dispensing of liquid phase 80a, sample dispenser 18 discards used dispensing chip 23a into an unillustrated disposal opening.

In Step S27, controller 45 determines whether or not the predetermined amount of liquid phase 80a is dispensed. To be more specific, controller 45 determines whether or not dispensing of the predetermined amount of liquid phase 80a using dispensing chip 23a is performed for a predetermined number of times. The predetermined number of times is twice, for example, and may be any number of times as long as liquid phase 80a is dispensed in an amount required for measurement into second reaction chamber 17. When the number of times of the dispensing is less than the predetermined number of times, controller 45 returns the processing to Step S23, and the processing of Steps S23 to S26 is repeated. More specifically, sample dispenser 18 repeats an operation for a predetermined number of times, including attaching new dispensing chip 23a, aspirating liquid phase 80a, dispensing liquid phase 80a, and discarding used dispensing chip 23a as a unit sequence.

Note that, when more than one second holder 52 is provided, another first reaction chamber 16 to be supplied for measurement of another sample can be provided other than second holder 52 configured to hold second reaction chamber 17 into which liquid phase 80a is dispensed. In this case, while repeating Steps S23 to S27, transfer unit 11 transfers another first reaction chamber 16 to reagent dispensing position 65 and first position 61.

In Step S28, transfer unit 11 transfers first reaction chamber 16 subjected to the aspiration and second reaction chamber 17 having liquid phase 80a dispensed therein to first position 61. In Step S29, transfer unit 27 transfers second reaction chamber 17 to reaction part 20a.

On the other hand, in Step S30, first reaction chamber 16 is discarded. To be more specific, transfer unit 27 transfers first reaction chamber 16 from first holder 51 to dispensing port 32a, and transfer unit 28 transfers first reaction chamber 16 from dispensing port 32a to relay unit 33. Then, transfer unit 29 transfers first reaction chamber 16 to from relay unit 33 inter-level transport unit 26, and chamber transport unit 41 discards first reaction chamber 16 by transporting first reaction chamber 16 from inter-level transport unit 26 to chamber disposal opening 42.

Upon completion of the processing of aspirating and dispensing liquid phase 80a containing immune complex 84, the processing advances to Step S10 of Fig. 14.

## Claims

1. An immune measuring apparatus (100) to measure a test substance in a sample by using antigen-antibody reaction, comprising:
a processing mechanism unit (10) adapted to perform, in a first reaction chamber (16), processing of forming an immune complex (84) containing the test substance (81) in the sample and a labeled substance (81) contained in a labeled reagent on a magnetic particle (82) contained in a solid-phase reagent and then liberating the immune complex from the magnetic particle with a liberating reagent;
a transfer unit (11) adapted to include a first holder to hold the first reaction chamber and a magnetic source, and transfer the first reaction chamber received in the first holder at a first position (61) to a second position (62) while magnetically collecting the magnetic particle in the first reaction chamber by the magnetic source such that a stop time for the magnetic collection of the magnetic particle is eliminated;
a dispenser (12) adapted to aspirate a liquid phase containing the liberated immune complex in the first reaction chamber transferred to the second position, and dispenses the liquid phase into a second reaction chamber (17); and
a detector (13) adapted to detect a signal based on a label contained in the immune complex in the liquid phase dispensed into the second reaction chamber.

2. The immune measuring apparatus (100) according to claim 1, wherein
the transfer unit (11) further includes a second holder adapted to hold the reaction chamber, and
the dispenser (12) is adapted to aspirate the liquid phase containing the immune complex liberated from the magnetic particle from the first reaction chamber set in the first holder, and dispense the aspirated liquid phase into the second reaction chamber set in the second holder.

3. The immune measuring apparatus (100) according to claim 2, wherein
the processing mechanism unit (10) includes a reagent dispenser (100)dispense a reagent into the reaction chamber,
the transfer unit (11) includes more than one second holder,
the reagent dispenser is adapted to dispense the reagent into the first reaction chamber set in one of the second holders, and
the dispenser (12) is adapted to dispense the liquid phase containing the immune complex liberated from the magnetic particle into the second reaction chamber set in another second holder.

4. The immune measuring apparatus (100) according to any one of claims 1 to 3, wherein
the processing mechanism unit (10) includes a sample dispenser that dispenses the sample into the first reaction chamber,
the dispenser (12) is the sample dispenser, and
the sample dispenser is adapted to dispense the sample into the first reaction chamber transferred to a sample dispensing position by the transfer unit, aspirate the liquid phase containing the immune complex from the first reaction chamber transferred to the second position, and dispense the liquid phase into the second reaction chamber transferred to the sample dispensing position.

5. The immune measuring apparatus (100) according to claim 4, wherein
the second position is the same as the sample dispensing position,
the transfer unit (11) is adapted to place the first reaction chamber at the sample dispensing position to allow the liquid phase containing the immune complex to be aspirated, and place the second reaction chamber at the sample dispensing position to allow the liquid phase containing the immune complex to be dispensed.

6. The immune measuring apparatus (100) according to claim 4 or 5, wherein
the sample dispenser is configured such that a dispensing chip is detachably attachable to a tip of the sample dispenser,
the sample dispenser is adapted to perform dispensing of the sample via a dispensing chip attached, and perform aspiration and dispensing of the liquid phase containing the immune complex liberated from the magnetic particle via another dispensing chip attached.

7. The immune measuring apparatus (100) according to any one of claims 1 to 6, wherein
the processing mechanism unit (10) includes a reaction part that reacts a specimen in the reaction chamber by heating the specimen, and
the transfer unit (11) is adapted to receive the first reaction chamber, in which the immune complex is liberated from the magnetic particle with the liberating reagent, from the reaction part at the first position, and transfer the received first reaction chamber to the second position.

8. The immune measuring apparatus (100) according to any one of claims 1 to 7, wherein
the magnetic source is provided on a side of or below the first holder.

9. The immune measuring apparatus (100) according to claim 8, wherein
the magnetic source includes a permanent magnet provided on the side of the first holder, and
the permanent magnet is arranged with a magnetization surface thereof facing toward the first holder, and extends along a longitudinal direction of the first reaction chamber set in the first holder.

10. The immune measuring apparatus (100) according to claim 9, wherein
a pair of the permanent magnets are provided, and
the pair of permanent magnets are arranged side by side in a direction perpendicular to the longitudinal direction of the first reaction chamber, while magnetization surfaces with different polarities of the permanent magnets face toward the first holder.

11. An immune measuring method of measuring a test substance in a sample by using antigen-antibody reaction, comprising:
forming, in a first reaction chamber, an immune complex containing the test substance in the sample and a labeled substance contained in a labeled reagent on a magnetic particle contained in a solid-phase reagent;
dispensing a liberating reagent to liberate the immune complex from the magnetic particle into the first reaction chamber;
transferring the first reaction chamber to a first position for magnetic collection of the magnetic particle by a magnetic source;
transferring the first reaction chamber from the first position to a second position, while magnetically collecting the magnetic particle in the first reaction chamber by the magnetic source such that a stop time for the magnetic collection of the magnetic particle is eliminated;
aspirating a liquid phase containing the liberated immune complex in the first reaction chamber at the second position in a state where the magnetic particle is magnetically collected;
dispensing the aspirated liquid phase into a second reaction chamber different from the first reaction chamber; and
detecting a signal based on a label contained in the immune complex in the liquid phase dispensed into the second reaction chamber.

12. The immune measuring method according to claim 11, further comprising:
holding the first reaction chamber by a second holder, and
aspirating the liquid phase containing the immune complex liberated from the magnetic particle from the first reaction chamber, and dispenses the aspirated liquid phase into the second reaction chamber set in the second holder.

13. The immune measuring method according to claim 12, wherein the second holder includes a plurality of holders, the method further comprising:
dispensing the reagent into the first reaction chamber set in one of the second holders, and
dispensing the liquid phase containing the immune complex liberated from the magnetic particle into the second reaction chamber set in another of the second holders.

14. The immune measuring method according to any one of claims 11 to 13, further comprising:
dispensing the sample into the first reaction chamber transferred to a sample dispensing position;
aspirating the liquid phase containing the immune complex from the first reaction chamber transferred to the second position; and
dispensing the liquid phase into the second reaction chamber transferred to the sample dispensing position.

15. The immune measuring method according to claim 14, wherein
the second position is the same as the sample dispensing position,
the first reaction chamber is placed at the sample dispensing position to allow the liquid phase containing the immune complex to be aspirated, and the second reaction chamber is placed at the sample dispensing position to allow the liquid phase containing the immune complex to be dispensed.

## Patentansprüche

1. Immunmessvorrichtung (100) zum Messen einer Testsubstanz in einer Probe durch Verwenden einer Antigen-Antikörper-Reaktion, umfassend:
eine Verarbeitungsmechanismuseinheit (10), die dafür angepasst ist, in einer ersten Reaktionskammer (16) ein Verarbeiten eines Bildens eines Immunkomplexes (84), der die Testsubstanz (81) in der Probe und eine gelabelte Substanz (81), die in einem gelabelten Reagenz auf einem magnetischen Partikel (82) enthalten ist, der in einem Festphasenreagenz enthalten ist, enthält, und dann ein Befreien des Immunkomplexes von dem magnetischen Partikel mit einem Befreiungsreagenz durchzuführen;
eine Transfereinheit (11), die dafür angepasst ist, einen ersten Halter, um die erste Reaktionskammer und eine magnetische Quelle zu halten, einzuschließen, und die in dem ersten Halter an einer ersten Position (61) empfangene erste Reaktionskammer zu einer zweiten Position (62) während des magnetischen Einsammelns des magnetischen Partikels in die erste Reaktionskammer durch die magnetische Quelle zu transferieren, sodass eine Stopzeit für das magnetische Einsammeln des magnetischen Partikels eliminiert wird;
einen Spender (12), der dafür angepasst ist, eine Flüssigphase, die den befreiten Immunkomplex enthält, in der ersten Reaktionskammer, die zu der zweiten Position transferiert wurde, zu aspirieren und die Flüssigphase in eine zweite Reaktionskammer (17) abgibt; und
einen Detektor (13), der dafür angepasst ist, ein Signal basierend auf einem Label zu detektieren, das in dem Immunkomplex in der Flüssigphase enthalten ist, die in die zweite Reaktionskammer abgegeben wurde.

2. Immunmessvorrichtung (100) nach Anspruch 1, wobei
die Transfereinheit (11) weiter einen zweiten Halter einschließt, der dafür angepasst ist, die Reaktionskammer zu halten, und
der Spender (12) dafür angepasst ist, die Flüssigphase, die den vom magnetischen Partikel befreiten Immunkomplex enthält, aus der in dem ersten Halter eingesetzten ersten Reaktionskammer zu aspirieren und die aspirierte Flüssigphase in die in dem zweiten Halter eingesetzte zweite Reaktionskammer abzugeben.

3. Immunmessvorrichtung (100) nach Anspruch 2, wobei
die Verarbeitungsmechanismuseinheit (10) einen Reagenzspender (100) zum Abgeben eines Reagenzes in die Reaktionskammer einschließt,
die Transfereinheit (11) mehr als einen zweiten Halter einschließt,
der Reagenzspender dafür angepasst ist, das Reagenz in die in einen der zweiten Halter eingesetzte erste Reaktionskammer abzugeben, und
der Spender (12) dafür angepasst ist, die Flüssigphase, die den von dem magnetischen Partikel befreiten Immunkomplex enthält, in die in einen anderen zweiten Halter eingesetzte zweite Reaktionskammer abzugeben.

4. Immunmessvorrichtung (100) nach einem der Ansprüche 1 bis 3, wobei
die Verarbeitungsmechanismuseinheit (10) einen Probenspender enthält, der die Probe in die erste Reaktionskammer abgibt,
der Spender (12) der Probenspender ist, und
der Probenspender dafür angepasst ist, die Probe in die durch die Transfereinheit zu einer Probenabgabeposition transferierte erste Reaktionskammer abzugeben, die Flüssigphase, die den Immunkomplex enthält, aus der zu der zweiten Position transferierten ersten Reaktionskammer zu aspirieren, und die Flüssigphase in die zu der Probenabgabeposition transferierte zweite Reaktionskammer abzugeben.

5. Immunmessvorrichtung (100) nach Anspruch 4, wobei
die zweite Position dieselbe wie die Probenabgabeposition ist,
die Transfereinheit (11) dafür angepasst ist, die erste Reaktionskammer an der Probenabgabeposition zu platzieren, um der Flüssigphase, die den Immunkomplex erhält, zu erlauben, aspiriert zu werden, und die zweite Reaktionskammer an der Probenabgabeposition zu platzieren, um der Flüssigphase, die den Immunkomplex enthält, zu erlauben, abgegeben zu werden.

6. Immunmessvorrichtung (100) nach Anspruch 4 oder 5, wobei
der Probenspender so eingerichtet ist, dass ein Abgabechip abnehmbar an einer Spitze des Probenspenders anbringbar ist,
der Probenspender dafür angepasst ist, das Abgeben der Probe über einen angebrachten Abgabechip und das Aspirieren und Abgeben der Flüssigphase, die den von dem magnetischen Partikel befreiten Immunkomplex enthält, über einen anderen angebrachten Abgabechip durchzuführen.

7. Immunmessvorrichtung (100) nach einem der Ansprüche 1 bis 6, wobei
die Verarbeitungsmechanismuseinheit (10) einen Reaktionsteil enthält, der einen Prüfling in der Reaktionskammer durch Erhitzen des Prüflings reagiert, und
die Transfereinheit (11) dafür angepasst ist, die erste Reaktionskammer, in der der Immunkomplex mit dem Befreiungsreagenz von dem magnetischen Partikel befreit wird, vom Reaktionsteil an der ersten Position zu empfangen und die empfangene erste Reaktionskammer zu der zweiten Position zu transferieren.

8. Immunmessvorrichtung (100) nach einem der Ansprüche 1 bis 7, wobei
die magnetische Quelle an einer Seite des oder unterhalb des ersten Halters bereitgestellt ist.

9. Immunmessvorrichtung (100) nach Anspruch 8, wobei
die magnetische Quelle einen Permanentmagneten einschließt, der an der Seite des ersten Halters bereitgestellt ist, und
der Permanentmagnet mit einer Magnetisierungsoberfläche von ihm dem ersten Halter zugewandt angeordnet ist und sich in Längsrichtung entlang der ersten Reaktionskammer, die in den ersten Halter eingesetzt ist, erstreckt.

10. Immunmessvorrichtung (100) nach Anspruch 9, wobei
ein Paar der Permanentmagneten bereitgestellt ist, und
das Paar von Permanentmagneten Seite an Seite in einer Richtung senkrecht zu der Längsrichtung der ersten Reaktionskammer angeordnet ist, während die Magnetisierungsoberflächen mit verschiedenen Polaritäten der Permanentmagneten dem ersten Halter zugewandt sind.

11. Immunmessverfahren zum Messen einer Testsubstanz in einer Probe durch Verwenden einer Antigen-Antikörper-Reaktion, umfassend:
Bilden, in einer ersten Reaktionskammer, eines Immunkomplexes, der die Testsubstanz in der Probe und eine gelabelte Substanz, die in einem gelabelten Reagenz auf einem magnetischen Partikel enthalten ist, der in einem Festphasenreagenz enthalten ist, enthält;
Abgeben eines Befreiungsreagenzes in die erste Reaktionskammer, um den Immunkomplex von dem magnetischen Partikel zu befreien;
Transferieren der ersten Reaktionskammer an eine erste Position zum magnetischen Einsammeln des magnetischen Partikels durch eine magnetische Quelle;
Transferieren der ersten Reaktionskammer von der ersten Position zu einer zweiten Position während des magnetischen Einsammelns des magnetischen Partikels in die erste Reaktionskammer durch die magnetische Quelle, sodass eine Stopzeit für das magnetische Einsammeln des magnetischen Partikels eliminiert wird;
Aspirieren einer Flüssigphase, die den befreiten Immunkomplex enthält, in die erste Reaktionskammer an der zweiten Position in einem Zustand, in dem das magnetische Partikel magnetisch eingesammelt ist;
Abgeben der aspirierten Flüssigphase in eine zweite Reaktionskammer, die von der ersten Reaktionskammer verschieden ist; und
Detektieren eines Signals basierend auf einem Label, das in dem Immunkomplex in der Flüssigphase enthalten ist, die in die zweite Reaktionskammer abgegeben wurde.

12. Immunmessverfahren nach Anspruch 11, weiter umfassend:
Halten der ersten Reaktionskammer durch einen zweiten Halter, und
Aspirieren der Flüssigphase, die den von dem magnetischen Partikel befreiten Immunkomplex enthält, aus der ersten Reaktionskammer, und Abgeben der aspirierten Flüssigphase in die zweite Reaktionskammer, die in den zweiten Halter eingesetzt ist.

13. Immunmessverfahren nach Anspruch 12, wobei der zweite Halter eine Vielzahl von Haltern enthält, das Verfahren weiter umfassend:
Abgeben des Reagenzes in die erste Reaktionskammer, die in einen der zweiten Halter eingesetzt ist, und
Abgeben der Flüssigphase, die den von dem magnetischen Partikel befreite Flüssigphase enthält, in die in einen anderen der zweiten Halter eingesetzte zweite Reaktionskammer.

14. Immunmessverfahren nach einem der Ansprüche 11 bis 13, weiter umfassend:
Abgeben der Probe in die in eine Probenabgabeposition transferierte erste Reaktionskammer;
Aspirieren der Flüssigphase, die den Immunkomplex enthält, aus der in die zweite Position transferierten ersten Reaktionskammer; und
Abgeben der Flüssigphase in die in die Probenabgabeposition transferierte zweite Reaktionskammer.

15. Immunmessverfahren nach Anspruch 14, wobei
die zweite Position dieselbe wie die Probenabgabeposition ist,
die erste Reaktionskammer an der Probenabgabeposition platziert wird, um der Flüssigphase, die den Immunkomplex erhält, zu erlauben, aspiriert zu werden, und die zweite Reaktionskammer an der Probenabgabeposition platziert wird, um der Flüssigphase, die den Immunkomplex enthält, zu erlauben, abgegeben zu werden.

## Revendications

1. Appareil de mesure immunitaire (100) pour mesurer une substance à tester dans un échantillon par l'utilisation d'une réaction antigène-anticorps, comprenant :
une unité de mécanisme de traitement (10) adaptée pour effectuer, dans une première chambre réactionnelle (16), un traitement de formation d'un complexe immunitaire (84) contenant la substance à tester (81) dans l'échantillon et une substance marquée (81) contenue dans un réactif marqué sur une particule magnétique (82) contenue dans un réactif en phase solide et ensuite la libération du complexe immunitaire à partir de la particule magnétique avec un réactif de libération ;
une unité de transfert (11) adaptée pour inclure un premier support pour contenir la première chambre réactionnelle et une source magnétique, et transférer la première chambre réactionnelle reçue dans le premier support à une première position (61) vers une seconde position (62) tout en collectant de manière magnétique la particule magnétique dans la première chambre réactionnelle par la source magnétique de sorte qu'un temps d'arrêt pour la collecte magnétique de la particule magnétique est éliminé ;
un distributeur (12) adapté pour aspirer une phase liquide contenant le complexe immunitaire libéré dans la première chambre réactionnelle transférée vers la seconde position, et distribuer la phase liquide dans une seconde chambre réactionnelle (17) ; et
un détecteur (13) adapté pour détecter un signal sur la base d'un marqueur contenu dans le complexe immunitaire dans la phase liquide distribuée dans la seconde chambre réactionnelle.

2. Appareil de mesure immunitaire (100) selon la revendication 1, dans lequel
l'unité de transfert (11) comprend en outre un second support adapté pour contenir la chambre réactionnelle, et
le distributeur (12) est adapté pour aspirer la phase liquide contenant le complexe immunitaire libéré à partir de la particule magnétique à partir de la première chambre réactionnelle installée dans le premier support, et distribuer la phase liquide aspirée dans la seconde chambre réactionnelle installée dans le second support.

3. Appareil de mesure immunitaire (100) selon la revendication 2, dans lequel
l'unité de mécanisme de traitement (10) comprend un distributeur de réactif (100) distribuant un réactif dans la chambre réactionnelle,
l'unité de transfert (11) comprend plus d'un second support,
le distributeur de réactif est adapté pour distribuer le réactif dans la première chambre réactionnelle installée dans l'un des seconds supports, et
le distributeur (12) est adapté pour distribuer la phase liquide contenant le complexe immunitaire libéré à partir de la particule magnétique dans la seconde chambre réactionnelle installée dans un autre second support.

4. Appareil de mesure immunitaire (100) selon l'une quelconque des revendications 1 à 3, dans lequel
l'unité de mécanisme de traitement (10) comprend un distributeur d'échantillon qui distribue l'échantillon dans la première chambre réactionnelle,
le distributeur (12) est le distributeur d'échantillon, et
le distributeur d'échantillon est adapté pour distribuer l'échantillon dans la première chambre réactionnelle transférée vers une position de distribution d'échantillon par l'unité de transfert, aspirer la phase liquide contenant le complexe immunitaire à partir de la première chambre réactionnelle transférée vers la seconde position, et distribuer la phase liquide dans la seconde chambre réactionnelle transférée vers la position de distribution d'échantillon.

5. Appareil de mesure immunitaire (100) selon la revendication 4, dans lequel
la seconde position est identique à la position de distribution d'échantillon,
l'unité de transfert (11) est adaptée pour placer la première chambre réactionnelle à la position de distribution d'échantillon pour permettre à la phase liquide contenant le complexe immunitaire d'être aspirée, et placer la seconde chambre réactionnelle à la position de distribution d'échantillon pour permettre à la phase liquide contenant le complexe immunitaire d'être distribuée.

6. Appareil de mesure immunitaire (100) selon la revendication 4 ou 5, dans lequel
le distributeur d'échantillon est configuré de façon qu'une puce de distribution puisse être fixée amovible à une extrémité du distributeur d'échantillon,
le distributeur d'échantillon est adapté pour effectuer la distribution de l'échantillon via une puce de distribution fixée, et effectuer l'aspiration et la distribution de la phase liquide contenant le complexe immunitaire libéré à partir de la particule magnétique via une autre puce de distribution fixée.

7. Appareil de mesure immunitaire (100) selon l'une quelconque des revendications 1 à 6, dans lequel
l'unité de mécanisme de traitement (10) comprend une partie de réaction qui fait réagir un spécimen dans la chambre réactionnelle en chauffant le spécimen, et
l'unité de transfert (11) est adaptée pour recevoir la première chambre réactionnelle, dans laquelle le complexe immunitaire est libéré à partir de la particule magnétique avec le réactif de libération, provenant de la partie de réaction à la première position, et transférer la première chambre réactionnelle reçue vers la seconde position.

8. Appareil de mesure immunitaire (100) selon l'une quelconque des revendications 1 à 7, dans lequel
la source magnétique est fournie d'un côté ou en dessous du premier support.

9. Appareil de mesure immunitaire (100) selon la revendication 8, dans lequel
la source magnétique comprend un aimant permanent fourni du côté du premier support, et
l'aimant permanent est agencé avec une surface d'aimantation de celui-ci en face du premier support, et s'étend le long d'une direction longitudinale de la première chambre réactionnelle installée dans le premier support.

10. Appareil de mesure immunitaire (100) selon la revendication 9, dans lequel
une paire des aimants permanents est fournie, et
les aimants permanents de la paire sont agencés côte à côte dans une direction perpendiculaire à la direction longitudinale de la première chambre réactionnelle, tandis que des surfaces d'aimantation ayant des polarités différentes des aimants permanents sont orientées vers le premier support.

11. Procédé de mesure immunitaire de mesure d'une substance à tester dans un échantillon par l'utilisation d'une réaction antigène-anticorps, comprenant :
la formation, dans une première chambre réactionnelle, d'un complexe immunitaire contenant la substance à tester dans l'échantillon et une substance marquée contenue dans un réactif marqué sur une particule magnétique contenue dans un réactif en phase solide ;
la distribution d'un réactif de libération pour libérer le complexe immunitaire à partir de la particule magnétique dans la première chambre réactionnelle ;
le transfert de la première chambre réactionnelle vers une première position pour la collecte magnétique de la particule magnétique par une source magnétique ;
le transfert de la première chambre réactionnelle à partir de la première position vers une seconde position, tout en collectant de manière magnétique la particule magnétique dans la première chambre réactionnelle par la source magnétique de sorte qu'un temps d'arrêt pour la collecte magnétique de la particule magnétique est éliminé ;
l'aspiration d'une phase liquide contenant le complexe immunitaire libéré dans la première chambre réactionnelle à la seconde position dans un état où la particule magnétique est collectée de manière magnétique ;
la distribution de la phase liquide aspirée dans une seconde chambre réactionnelle différente de la première chambre réactionnelle ; et
la détection d'un signal sur la base d'une étiquette contenue dans le complexe immunitaire dans la phase liquide distribuée dans la seconde chambre réactionnelle.

12. Procédé de mesure immunitaire selon la revendication 11, comprenant en outre :
le maintien de la première chambre réactionnelle par un second support, et
l'aspiration de la phase liquide contenant le complexe immunitaire libéré à partir de la particule magnétique à partir de la première chambre réactionnelle, et la distribution de la phase liquide aspirée dans la seconde chambre réactionnelle installée dans le second support.

13. Procédé de mesure immunitaire selon la revendication 12, dans lequel le second support comprend une pluralité de supports, le procédé comprenant en outre :
la distribution du réactif dans la première chambre réactionnelle installée dans l'un des seconds supports, et
la distribution de la phase liquide contenant le complexe immunitaire libéré à partir de la particule magnétique dans la seconde chambre réactionnelle installée dans un autre des seconds supports.

14. Procédé de mesure immunitaire selon l'une quelconque des revendications 11 à 13, comprenant en outre :
la distribution de l'échantillon dans la première chambre réactionnelle transférée à une position de distribution d'échantillon ;
l'aspiration de la phase liquide contenant le complexe immunitaire à partir de la première chambre réactionnelle transférée à la seconde position ; et
la distribution de la phase liquide dans la seconde chambre réactionnelle transférée à la position de distribution d'échantillon.

15. Procédé de mesure immunitaire selon la revendication 14, dans lequel
la seconde position est identique à la position de distribution d'échantillon,
la première chambre réactionnelle est placée à la position de distribution d'échantillon pour permettre à la phase liquide contenant le complexe immunitaire d'être aspirée, et la seconde chambre réactionnelle est placée à la position de distribution d'échantillon pour permettre à la phase liquide contenant le complexe immunitaire d'être distribuée.
